# EUROPÄISCHE PATENTSCHRIFT

(11) **EP 2 335 638 B1**
(45) Veröffentlichungstag und Bekanntmachung des Hinweises auf die Patenterteilung: **16.11.2011**
(21) Anmeldenummer: 09178643.4
(22) Anmeldetag: 10.12.2009
(51) Int. Cl.: A61C 5/06, B05C 17/01

(54) **Medizinische, insbesondere dentale, Behandlungsvorrichtung zur Abgabe eines Mediums**
Medical, in particular dental, treatment device for dispensing a medium
Dispositif de traitement médical en particulier dentaire pour délivrer une substance

(43) Veröffentlichungstag der Anmeldung: 22.06.2011
(73) Patentinhaber: W & H Dentalwerk Bürmoos GmbH, 5111 Bürmoos (AT)
(72) Erfinder: Wagner, Hannes, 5023, Salzburg (AT); Schuh, Walter, 5111, Bürmoos (AT)
(74) Vertreter: Benda, Ralf

(56) Entgegenhaltungen:
- EP-A1- 1 923 018
- EP-A1- 2 140 829
- WO-A1-2004/082508
- WO-A1-2009/113843
- US-A1- 2009 202 961

## Beschreibung

Die vorliegende Erfindung betrifft eine medizinische, insbesondere dentale, Behandlungsvorrichtung zur Abgabe eines Mediums nach dem Oberbegriff des Anspruchs 1.

Eine derartige Behandlungsvorrichtung ist aus dem Patent US 7,014,462 B1 und der Offenbarung WO 2009 113843 bekannt. Der Nachteil dieser Behandlungsvorrichtung liegt darin, dass die Behandlungsvorrichtung dem Anwender bei der praktischen Arbeit keine oder eine nur sehr geringe Flexibilität in Bezug auf unterschiedliche Anwendungen oder unterschiedliche abzugebende Medien bietet.

Der vorliegenden Erfindung liegt daher die Aufgabe zugrunde eine medizinische, insbesondere dentale, Behandlungsvorrichtung zur Abgabe eines Mediums zu schaffen, die dem Anwender mehr Möglichkeiten eröffnet, Betriebsparameter für spezifische Anwendungen auszuwählen oder die Behandlungsvorrichtung auf Medien mit unterschiedlichen Eigenschaften zu konfigurieren.

Erfindungsgemäss weist die erfindungsgemäße medizinische, insbesondere dentale, Behandlungsvorrichtung zur Abgabe eines Mediums ein Basisteil mit einer Energieversorgungseinheit und einer elektrischen oder elektronischen Steuervorrichtung und zumindest ein mit dem Basisteil über eine Kupplungsvorrichtung lösbar verbindbares Förderteil auf, das eine Schallquelle, insbesondere eine Ultraschallquelle, und eine Fördervorrichtung zur Abgabe des Mediums von dem Förderteil umfasst. Gemäß einem bevorzugten Ausführungsbeispiel weist die Behandlungsvorrichtung mehrere Förderteile auf, die insbesondere unterschiedliche Schallquellen umfassen. Die unterschiedlichen Schallquellen erzeugen unterschiedliche Schwingungsamplituden, Schwingungsfrequenzen oder geben unterschiedliche Leistungen ab, so dass der Anwender je nach Anwendung oder abzugebendem Medium das Förderteil mit der passenden Schallquelle an das Basisteil anschließen kann.

Gemäß einem alternativen Ausführungsbeispiel weist die Behandlungsvorrichtung zumindest ein mit dem Basisteil über die Kupplungsvorrichtung lösbar verbindbares Wirkteil auf, das anstelle des Förderteils an das Basisteil anschließbar ist. Das zumindest eine Wirkteil weist (im Gegensatz zu dem Förderteil) keine Fördervorrichtung zur Abgabe eines Mediums auf. Bevorzugt werden zumindest ein Förderteil und zumindest ein Basisteil in einer Anwendung gemeinsam, jedoch zeitlich aufeinander folgend verwendet. Gemäß einem besonders bevorzugten Ausführungsbeispiel wird das Wirkteil zum Einwirken auf das vom Förderteil abgegebene Medium verwendet. Unter Einwirken auf das Medium wird insbesondere eine chemische, physikalische, thermische, mechanische, elektromagnetische Einwirkung, Bearbeitung oder Aktivierung verstanden.

Erfindungsgemäß ist die Behandlungsvorrichtung als kabelloses Handstück und die Energieversorgungseinheit als Batterie oder Akkumulator ausgebildet.

Gemäß einem anderen Ausführungsbeispiel weist das Basisteil einen, insbesondere pistolenförmigen, Griffabschnitt auf, in dem zumindest eine Teil der Energieversorgungseinheit und / oder der elektrischen oder elektronischen Steuervorrichtung aufgenommen ist. Alternativ ist die Behandlungsvorrichtung als gerades oder als gewinkeltes Handstück ausgebildet, das einen Basisteil und zumindest einen Förderteil aufweist, so wie dies im Vorherstehenden beschrieben ist.

Die Schallquelle, insbesondere Ultraschallquelle, ist zum Beispiel als pneumatische Schallquelle, als piezoelektrische Schallquelle oder als magnetostriktive Schallquelle ausgebildet.

Die Fördervorrichtung weist zum Beispiel einen beweglichen oder verschiebbaren Kolben sowie ein Bedienelement zum selektiven Bewegen des Kolbens auf. Das Bedienelement ist insbesondere als mechanisches Bedienelement, zum Beispiel als bewegbarer oder verschwenkbarer Hebel, oder als elektrisch betreibbares Bedienelement, zum Beispiel als Taster, ausgebildet.

Die von der Behandlungsvorrichtung förder- und abgebbaren Medien umfassen zum Beispiel ein Füllmaterial zur Abgabe in eine, insbesondere dentale, Kavität, ein Füllmaterial zur Abgabe in eine Zahnwurzel, ein Spül- oder Desinfektionsmittel zur Abgabe in eine Zahnwurzel oder ein Anästhetikum zur intraossären Abgabe, insbesondere in einen Kieferknochen.

Gemäß einem Ausführungsbeispiel weist das zumindest eine Förderteil eine Aufnahme für das Medium oder einen Anschluss für einen Medienbehälter und eine Sonotrode zur Übertragung der von der Schallquelle, insbesondere der Ultraschallquelle, erzeugten Schwingungen auf das Medium, die Aufnahme und / oder den Anschluss auf. Damit wird in vorteilhafter Weise eine besonders gute Förderbarkeit, Verteilung des Mediums im Zielgebiet oder Aktivierung des Mediums erzielt.

Gemäß einem Ausführungsbeispiel weist das zumindest eine Förderteil eine Strahlungsquelle zur Abgabe einer das Medium aktivierenden Strahlung auf. Gemäß einem anderen Ausführungsbeispiel umfasst das zumindest eine Förderteil ein Werkzeug für das Medium, insbesondere eine Modelliersonde, eine intraossäre Nadel oder eine Wurzelkanalsonde, und eine Sonotrode zur Übertragung der von der Schallquelle, insbesondere der Ultraschallquelle, erzeugten Schwingungen auf das Werkzeug. Gemäß einem weiteren Ausführungsbeispiel ist das zumindest eine Förderteil mit einer Wärmequelle zur Erwärmung des Mediums versehen. Diese Ausführungsbeispiele erhöhen die Funktionalität der Behandlungsvorrichtung und die Bedienerfreundlichkeit, da der Anwender mehrere Behandlungsschritte mit einem Förderteil durchführen kann.

Wie im Vorherstehenden bereits beschrieben weist die Behandlungsvorrichtung gemäß einem Ausführungsbeispiel zumindest ein mit dem Basisteil über die Kupplungsvorrichtung lösbar verbindbares Wirkteil auf, das anstelle des Förderteils an das Basisteil anschließbar ist. Der Vorteil dieses Ausführungsbeispiels liegt darin, dass insbesondere durch die Verwendung mehrerer unterschiedlicher Wirkteile die Funktionalität der Behandlungsvorrichtung weiter erhöht wird. So können zum Beispiel mehrere Wirkteile mit unterschiedlichen Strahlungsquellen zur Verfügung gestellt werden, wobei jede Strahlungsquelle eine andere Strahlung emittiert, zum Beispiel mit jeweils einer spezifischen Wellenlänge und / oder Leistung. Alternativ kann zum Beispiel mittels mehrerer Wirkteile, die jeweils eine Modelliersonde mit einem individuell geformten Modellierabschnitt aufweisen, auf Zähne abgegebenes Zahnfüllmaterial je nach Erfordernis modelliert und / oder verdichtet werden.

Gemäß einem Ausführungsbeispiel ist das zumindest eine Wirkteil dazu ausgebildet, auf das vom Förderteil abgegebene Medium einzuwirken. Das zumindest eine Wirkteil umfasst dazu zum Beispiel eine Strahlungsquelle zur Abgabe einer das Medium aktivierenden Strahlung und / oder ein Werkzeug für das abgegebene Medium, insbesondere eine Modelliersonde oder eine Obturiernadel, und / oder eine Schallquelle, insbesondere eine Ultraschallquelle, zur Übertragung von Schwingungen auf das Werkzeug und / oder eine Wärmequelle zur Erwärmung des Mediums.

Gemäß einem alternativen Ausführungsbeispiel ist das zumindest eine Wirkteil dazu ausgebildet, auf die Behandlungsstelle einzuwirken, auf die das Förderteil das Medium abgegeben hat oder abgeben wird. Das zumindest eine Wirkteil umfasst dazu zum Beispiel ein Werkzeug zur Wurzelkanalaufbereitung und bevorzugt eine Schallquelle, insbesondere eine Ultraschallquelle, zur Übertragung von Schwingungen auf das Werkzeug.

Die medizinische, insbesondere dentale, Behandlungsvorrichtung kann für viele unterschiedliche Anwendungen verwendet werden, zum Beispiel:
- zur Abgabe von Füllmaterial in eine, insbesondere dentale, Kavität;
- zum Einwirken auf das vom Förderteil in die Kavität abgegebenen Füllmaterial, insbesondere zum Bestrahlen des Füllmaterials und / oder zum Verdichten und / oder Modellieren des Füllmaterials;
   wobei bevorzugt bei beiden im Vorherstehenden angeführten Anwendungen ein Füllmaterial verwendet wird, bei dem durch das Beaufschlagen mit Schall oder Ultraschall und / oder Wärme eine Viskositätsänderung erzielt wird, zum Beispiel ein Zahnzement oder eine Füllmasse auf Kunstharzbasis, bevorzugt eine Füllmasse auf Kunstharzbasis, die durch das Einwirken von Licht polymerisierbar oder aushärtbar ist, insbesondere eine hochviskose, einen hohen Anteil an anorganischen Füllstoffen aufweisende dentalen Füllmasse auf Kunstharzbasis.
- zur Abgabe von Füllmaterial in eine Zahnwurzel;
- zum Einwirken auf das vom Förderteil in die Zahnwurzel abgegebene Füllmaterial, insbesondere zum Erwärmen und / oder zum Verdichten und / oder Modellieren des Füllmaterials;
   wobei bevorzugt bei den beiden letztgenannten Anwendungen ein Füllmaterial verwendet wird, bei dem durch das Beaufschlagen mit Schall oder Ultraschall und / oder Wärme eine Viskositätsänderung erzielt wird, zum Beispiel ein Zahnzement oder Gutta Percha.
- zur Abgabe eines Spül- oder Desinfektionsmittels in eine Zahnwurzel unter zusätzlicher Verwendung einer Wurzelkanalsonde; alternativ kann für diese Anwendung eine medizinische, insbesondere dentale, Behandlungsvorrichtung verwendet werden, die eine Schallquelle, insbesondere eine Ultraschallquelle, eine Fördervorrichtung zur Abgabe eines Mediums und eine Wurzelkanalsonde umfasst;
   wobei für diese letztgenannte Anwendung die Wurzelkanalsonde bevorzugt als hohle, mit der Fördervorrichtung verbindbare Sonde ausgebildet ist und besonders bevorzugt von der Schallquelle, insbesondere der Ultraschallquelle, erzeugte Schwingungen direkt oder indirekt, insbesondere über die Wurzelkanalsonde, auf das Spül- oder Desinfektionsmittel übertragen werden, um das Spül- oder Desinfektionsmittel zu aktivieren.
- zum Erweitern eines Zahnwurzelkanals; alternativ kann für diese Anwendung eine medizinische, insbesondere dentale, Behandlungsvorrichtung verwendet werden, die eine Schallquelle, insbesondere eine Ultraschallquelle, umfasst;
   wobei für diese letztgenannte Anwendung die Behandlungsvorrichtung bevorzugt eine Dentalfeile aufweist, insbesondere eine diamantierte Dentalfeile.
- zur intraossären Abgabe eines Anästhetikums, insbesondere in einen Kieferknochen, unter zusätzlicher Verwendung einer intraossären Nadel; alternativ kann für diese Anwendung eine medizinische, insbesondere dentale, Behandlungsvorrichtung verwendet werden, die eine Schallquelle, insbesondere eine Ultraschallquelle, eine Fördervorrichtung zur Abgabe eines Mediums und eine intraossäre Nadel umfasst.

Die einzelnen angeführten Verwendungen können entweder mittels eines Förderteils und / oder mittels eines Wirkteils durchgeführt werden.

Bevorzugt können mehrere der angeführten Verwendungen kombiniert werden, um eine besonders zeit- und kosteneffektive Behandlung zu erzielen. Besonders bevorzugt werden während einer derartigen Behandlung zwei oder mehr Förderteile oder zumindest ein Förderteil und zumindest ein Wirkteil aufeinander folgend verwendet. Ein Ausführungsbeispiel einer derartigen kombinierten Verwendung ist wie folgt ausgestaltet: Zuerst wird an das Basisteil ein Förderteil mit einer Schallquelle und einer Fördervorrichtung angeschlossen, mittels dem Füllmaterial in eine dentale Kavität abgegeben wird. Anschließend wird das Förderteil vom Basisteil getrennt und letzteres mit einem erstes Wirkteil mit einer, insbesondere mit einer Schallquelle verbundenen, Modelliersonde verbunden, welches auf das vom Förderteil in die Kavität abgegebenen Füllmaterial einwirkt, indem es das Füllmaterial modelliert und / oder verdichtet. Nach der Verdichtung oder Modellierung wird das erste Wirkteil vom Basisteil gelöst und das Basisteil mit einem zweiten Wirkteil mit einer Strahlungsquelle verbunden, um das Füllmaterial zu bestrahlen und auszuhärten. Alternativ ist nur ein Wirkteil vorgesehen, welches eine, insbesondere mit einer Schallquelle verbundenen, Modelliersonde und eine Strahlungsquelle umfasst.

Ein anderes Ausführungsbeispiel einer Kombination mehrerer der oben angeführten Verwendungen der Behandlungsvorrichtung für eine dentale Wurzelkanalbehandlung umfasst folgende Schritte: Mittels eines ersten, mit dem Basisteil verbundenen Förderteils, das mit einer intraossären Nadel versehen ist und eine mit der Nadel verbundene Schallquelle aufweist, wird durch die Übertragung der Schwingungen der Schallquelle auf die Nadel eine Bohrung in ein Kiefer gesetzt. Anschließend wird mit der Fördervorrichtung des ersten Förderteils ein Anästhetikum über die hohle intraossäre Nadel in die Bohrung und das Kiefer abgegeben. Anschließend wird das erste Förderteil vom Basisteil getrennt. In einem folgenden Schritt wird ein erstes Wirkteil mit dem Basisteil verbunden, wobei das erste Wirkteil eine Schallquelle und ein damit verbundenes Werkzeug zur Wurzelkanalaufbereitung aufweist, insbesondere eine diamantierte Feile. Damit wird ein zuvor geöffneter Zahnwurzelkanal erweitert. In einem weiteren Schritt wird das Basisteil mit einem zweiten Förderteil verbunden, das mit einer Wurzelkanalsonde versehen ist und das eine Schallquelle und eine Fördervorrichtung für ein Spül- oder Desinfektionsmittel aufweist. Mit Hilfe der Fördervorrichtung wird das Spül- oder Desinfektionsmittel in den Wurzelkanal geleitet und mittels der von der Schallquelle erzeugten und von der Wurzelkanalsonde übertragenen Schwingungen aktiviert. Im Folgenden wird ein drittes Förderteil an das Basisteil angeschlossen, das eine Fördervorrichtung, eine Schallquelle und optional eine Wärmequelle aufweist und das dazu dient, ein Füllmaterial, insbesondere Gutta Percha, in den Wurzelkanal zu fördern. Abschließend wird an das Basisteil ein weiteres Wirkteil angeschlossen, das eine Wärmequelle aufweist und ein Werkzeug aufweist, um das in den Wurzelkanal abgegebene Gutta Percha zu verteilen, zu verdichten oder zu modellieren, und / oder es wird ein weiteres Wirkteil mit dem Basisteil verbunden, das ein Werkzeug, zum Beispiel eine Obturiernadel, und eine Schallquelle umfasst, so dass die auf die Obturiernadel übertragenen Schwingungen das Gutta Percha verteilen, verdichten oder modellieren oder es wird an das Basisteil ein Wirkteil angeschlossen, das eine Wärmequelle und ein Werkzeug und eine mit dem Werkzeug verbundenen Schallquelle umfasst, um das Gutta Percha zu verteilen, verdichten oder zu modellieren. Selbstverständlich ist es auch möglich ein Förderteil derart zu gestalten, dass es unterschiedliche Medien abgegeben kann, so dass zum Beispiel in der im Vorstehenden beschriebenen Anwendung anstelle der drei Förderteile nur zwei oder nur ein Förderteil benötigt wird.

Die Erfindung wird nachfolgend anhand bevorzugter Ausführungsbeispiele und Bezug nehmend auf die beigefügten Zeichnungen erläutert:
Figur 1 zeigt eine Außenansicht eines Ausführungsbeispiels einer medizinischen, insbesondere dentalen, kabellosen Behandlungsvorrichtung zur Abgabe eines Mediums mit einem Basisteil und einem Förderteil.
Figur 2 zeigt eine Schnittdarstellung durch den Basisteil der Behandlungsvorrichtung der Figur 1.
Figur 3 zeigt ein Ausführungsbeispiel eines Basisteils mit der Schnittstelle zum Anschluss eines Förderteils oder eines Wirkteils.
Figur 4 zeigt ein Ausführungsbeispiel eines Förderteils mit der Schnittstelle zum Anschluss an das Basisteil.
Figur 5 zeigt eine Außenansicht eines Ausführungsbeispiels eines Förderteils.
Figuren 6 - 9 zeigen Schnittdarstellungen durch vier unterschiedliche Ausführungsbeispiele von Förderteilen mit daran angeschlossenen Medienbehältern.
Figur 10 zeigt eine Außenansicht eines Ausführungsbeispiels einer medizinischen, insbesondere dentalen, Behandlungsvorrichtung zur Abgabe eines Mediums mit einem Basisteil und einem Wirkteil.
Figur 11 zeigt eine Schnittdarstellung durch das Wirkteil der Behandlungsvorrichtung der Figur 10.
Figur 12 zeigt eine Außenansicht eines alternativen Ausführungsbeispiels einer medizinischen, insbesondere dentalen, Behandlungsvorrichtung zur Abgabe eines Mediums mit einem Basisteil und einem Wirkteil.
Figur 13 zeigt eine Schnittdarstellung durch das Wirkteil der Behandlungsvorrichtung der Figur 12.
Figuren 14 - 16 zeigen Schnittdarstellungen durch drei unterschiedliche Ausführungsbeispiele von Wirkteilen.

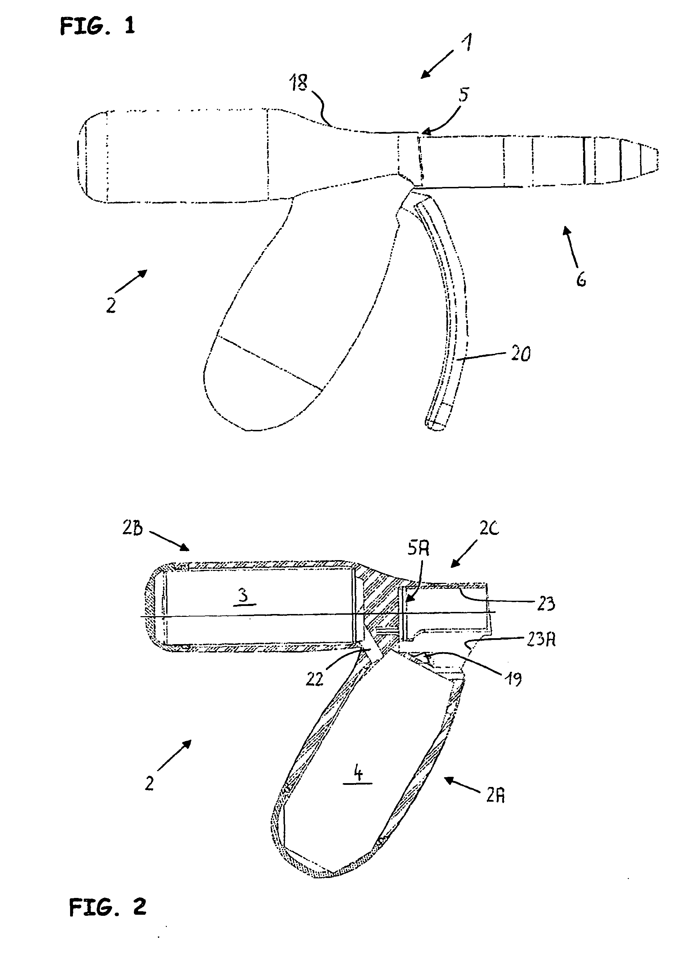

Die in den Figuren 1 - 16 dargestellte Behandlungsvorrichtung 1 ist als kabelloses, pistolenförmiges Handstück ausgebildet, das eine Außenhülse 18, einen Basisteil 2 und mehrere mit dem Basisteil 2 verbindbare Förderteile 6 - 6E und Wirkteile 13A - 13E umfasst. Die Verbindung zwischen dem Basisteil 2 und den Förderteilen 6 - 6E oder den Wirkteilen 13A - 13E erfolgt über eine Kupplungsvorrichtung 5. Die Kupplungsvorrichtung 5 umfasst am Basisteil 2 und an den Förderteilen 6 - 6E oder den Wirkteilen 13A - 13E vorgesehene, miteinander verbindbare Kupplungselemente 5A, 5B.

Das Basisteil 2 umfasst einen Griffabschnitt 2A und einen gewinkelt dazu angeordneten Fortsatz 2B, die über einen Mittelabschnitt 2C miteinander verbunden sind. Der Griffabschnitt 2A ist hohl ausgebildet und nimmt in seinem Inneren eine elektrische oder elektronische Steuervorrichtung 4 auf, die insbesondere zumindest ein elektrisches oder elektronisches Bauteil umfasst, zum Beispiel einen Schaltkreis, einen Mikrocomputer, eine Platine oder ein Speicherelement. Die Steuervorrichtung 4 dient zur Steuerung und / oder Regelung der Behandlungsvorrichtung 1 und der Betriebsparameter und / oder zur Speicherung von Betriebs- oder Behandlungsdaten, Betriebs- oder Behandlungsabläufen oder Reinigungsdaten. Gemäß einem Ausführungsbeispiel ist die Steuervorrichtung 4 dazu ausgebildet, die an das Basisteil anschließbaren Förderteile 6 - 6E oder Wirkteile 13A - 13E zu erkennen und insbesondere für das jeweilige angeschlossene Förderteil 6 - 6E oder Wirkteil 13A - 13E zumindest einen spezifischen Betriebsparameter auszuwählen und das angeschlossene Förderteil 6 - 6E oder Wirkteil 13A - 13E gemäß diesem Betriebsparameter zu betreiben.

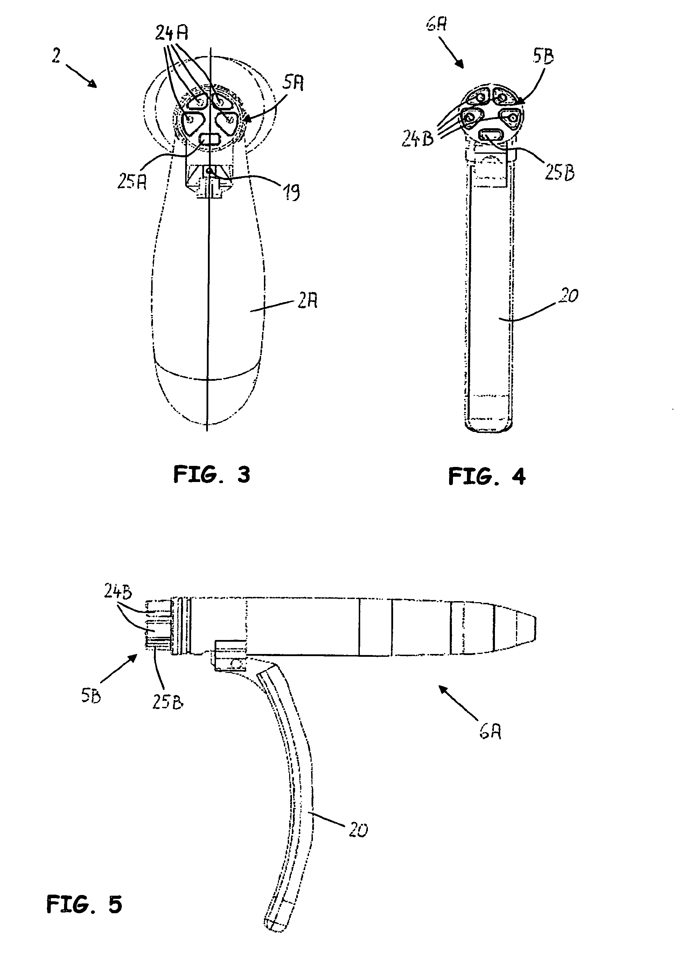

Gemäß einem anderen Ausführungsbeispiel ist die Steuervorrichtung 4 dazu ausgebildet, mit einem oder mehreren an der Behandlungsvorrichtung 1 vorgesehenen Stellmitteln und / oder Anzeigevorrichtungen zusammenzuwirken und einen uni- oder bidirektionalen Signal- oder Datenaustausch zwischen der Steuervorrichtung 4 und den Stellmitteln und / oder den Anzeigevorrichtungen zu bewerkstelligen. Die Steuervorrichtung ist dazu zum Beispiel mit einem Schaltelement 19 (Figuren 2, 3) verbunden, das über ein mechanisches oder elektrisches Stellelement, zum Beispiel einen Hebel 20, einen Drücker 21 oder einen Taster, geschaltet wird. Durch die Betätigung des Stellelements wird zum Beispiel die Behandlungsvorrichtung 1 aktiviert oder deaktiviert.

Der Fortsatz 2B des Basisteils 2 ist ebenfalls hohl ausgebildet und nimmt eine Energieversorgungseinheit 3 auf, zum Beispiel einen Akkumulator oder eine Batterie. Um Zugang zur Energieversorgungseinheit 3 zu erhalten, weist der Fortsatz 2B einen Deckel auf, der vom übrigen Fortsatz lösbar ist.

Die Energieversorgungseinheit 3 des Fortsatzes 2B und die Steuervorrichtung 4 des Griffabschnitts 2A sind über elektrische Leitungen miteinander verbunden, wobei die Leitungen in einem Leitungskanal 22 verlaufen, der die Innenräume des Fortsatzes 2B und des Griffabschnitts 2A verbindet.

Der Mittelabschnitt 2C des Basisteils 2 weist eine Aufnahme 23 auf, in der zumindest ein Teil eines Förderteils 6 - 6E und / oder eines Wirkteiles 13A - 13E aufnehmbar ist, insbesondere jener Teil eines Förderteils 6 - 6E und / oder eines Wirkteiles 13A - 13E, an dem das Kupplungselement 5B vorgesehen ist. Bevorzugt ist die Aufnahme 23 des Weiteren so ausgebildet, dass ein Teil des Hebels 20 oder des Drückers 21 oder eines damit operativ verbundenen Bauteils ebenfalls darin aufnehmbar ist, um das in dem Basisteil 2 angeordneten Schaltelement 19 zu betätigen. Die Aufnahme 23 umfasst eine Aufnahmeöffnung 23A für ein Förderteil 6 - 6E und / oder eine Wirkteil 13A - 13E und zumindest eine Wand, an der das Kupplungselement 5A des Basisteils 2 angeordnet ist. Insbesondere ist das Kupplungselement 5A an der der Aufnahmeöffnung 23 gegenüberliegenden Wand vorgesehen.

Das Kupplungselement 5A umfasst mehrere elektrische Kontakte 24A, zum Beispiel vier elektrische Kontakte, die mit der Energieversorgungseinheit 3 verbunden sind. Jeweils zwei dieser Kontakte 24A dienen zur Versorgung eines elektrischen Verbrauchers eines Förderteils 6 - 6E oder eines Wirkteils 13A - 13E, zum Beispiel einer elektrisch betriebenen Schall- oder Ultraschallquelle 7, 17, einer elektrisch betriebenen Wärmequelle 16 oder einer elektrisch betriebenen Licht- oder Strahlungsquelle 14. An jedem Kupplungselement 5B eines Förderteil 6 - 6E oder eines Wirkteil 13A - 13E sind dem entsprechend mehrere, insbesondere vier, elektrische Gegenkontakte 24B vorgesehen, die, wenn die Kupplungselement 5A, 5B miteinander gekuppelt werden, die Kontakte 24A kontaktieren und über elektrische Leitungen den Stromkreis zwischen der Energieversorgungseinheit 3 und den elektrischen Verbrauchern schließen. Die Kupplungsvorrichtung 5 ist bevorzugt als Steckkupplung mit Fortsätzen oder Stiften an einem Kupplungselement 5A, 5B und Aufnahmen oder Buchsen für die Fortsätze am anderen Kupplungselement 5A, 5B ausgebildet, wobei besonders bevorzugt die elektrischen Kontakte 24A, 24B die Fortsätze, Stifte, Aufnahmen oder Buchsen bilden oder darin angeordnet sind. Selbstverständlich kann die Kupplungsvorrichtung 5 jedoch auch andere bekannte Kupplungen, zum Beispiel Bajonett- oder Schraubkupplungen umfassen.

Des Weiteren ist an der Kupplungsvorrichtung 5 eine Verdrehsicherung vorgesehen, die eine Drehung des mit dem Basisteil 2 verbundenen Förderteils 6 - 6E oder eines Wirkteils 13A - 13E relativ zum Basisteil 2 verhindert. Die Verdrehsicherung ist bevorzugt als Steckvorrichtung ausgebildet, die insbesondere einen Fortsatz 25B und einen Rücksprung 25A zur Aufnahme des Fortsatzes 25B aufweist. Der Fortsatz 25B und der Rücksprung 25A sind jeweils auf einem der beiden Kupplungselemente 5A, 5B vorgesehen.

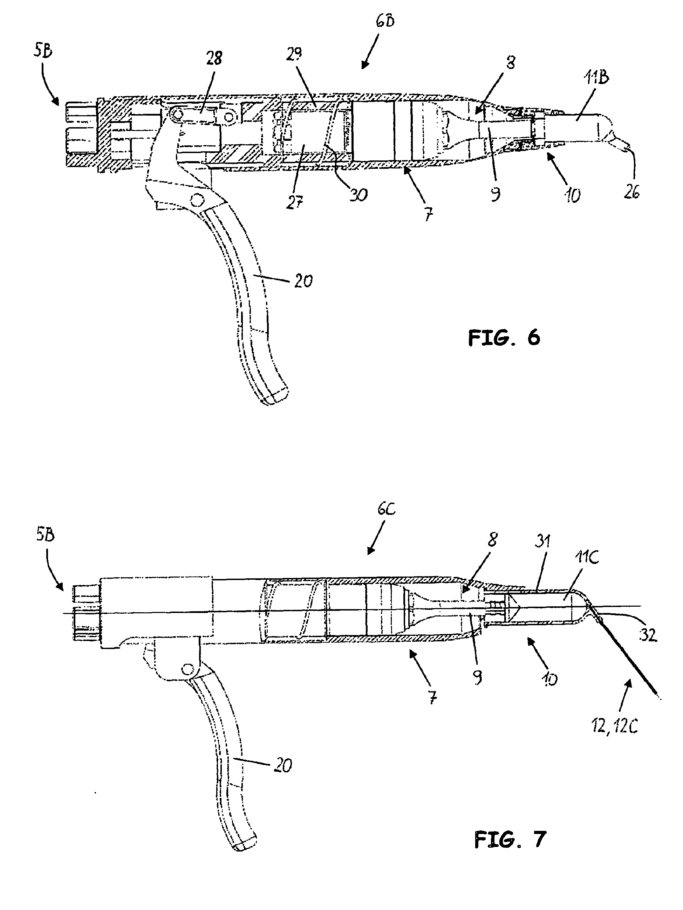

Die in den Figuren 6 - 9 dargestellten Förderteile 6B - 6E umfassen jeweils eine Schallquelle oder Ultraschallquelle 7, die insbesondere als piezoelektrische, magnetostriktive oder pneumatische Schallquelle 7 ausgebildet ist, eine Fördervorrichtung 8 zur Abgabe eines Mediums von den Förderteilen 6B - 6E, eine Aufnahme für das Medium oder einen Anschluss 10 für einen Medienbehälter 11B - 11E und eine Sonotrode 9 zur Übertragung der von der Schallquelle 7 erzeugten Schwingungen auf das Medium, die Aufnahme und / oder den Anschluss 10.

Je nach vorgesehener Anwendung weisen die Förderteile 6B - 6E unterschiedliche Merkmale auf oder sind bestimmte Bauteile der verschiedenen Förderteile 6B - 6E spezifisch ausgebildet:
Das in Figur 6 dargestellt Förderteil 6B wird zur Abgabe von Füllmaterial in eine dentale Kavität verwendet, insbesondere von Füllmaterial bei dem durch das Beaufschlagen mit Schall oder Ultraschall und / oder Wärme eine Viskositätsänderung erzielt wird, zum Beispiel ein Zahnzement oder eine Füllmasse auf Kunstharzbasis, bevorzugt eine Füllmasse auf Kunstharzbasis, die durch das Einwirken von Licht polymerisierbar oder aushärtbar ist, insbesondere eine hochviskose, einen hohen Anteil an anorganischen Füllstoffen aufweisende dentalen Füllmasse auf Kunstharzbasis. Die Viskositätsänderung wird hierbei durch die Übertragung der von der Schallquelle 7 erzeugten Schwingungen über die Sonotrode 9 auf das Füllmaterial erzielt.

Das Füllmaterial ist in einem Medienbehälter 11B enthalten, der in die Aufnahme 10 des Förderteils 6B eingesteckt ist. Bevorzugt werden die von der Schallquelle 7 erzeugten Schwingungen direkt oder indirekt, zum Beispiel über das Füllmaterial, auch auf den Medienbehälter 11B, insbesondere auf dessen Abgabedüse 26, übertragen. Die Aufnahme 10 ist bevorzugt vom Förderteil 6B lösbar.

Die Schallquelle 7 umfasst mehrere piezoelektrische Elemente, die an einem Ende mit der Sonotrode 9 und an einem anderen Ende mit einer Schwing- oder Gegenmasse 27 verbunden. Die Gegenmasse 27 ist von einer Schiebehülse 29 umgeben.

Die Fördervorrichtung 8 umfasst die verschiebbar im Förderteil 6B angeordnete Sonotrode 9 und das, insbesondere als Hebel 20 ausgebildete, Bedienelement, mittels dem die Sonotrode 9 innerhalb des Förderteils 6B und relativ zur Aufnahme 10 verschoben werden kann. Der Hebel 20 ist drehbar am Förderteil 6B befestigt und weist an seinem im Inneren der Förderteils 6B angeordneten Ende einen Mitnehmer 28 auf, der bei Betätigung des Hebels 20 die Schiebehülse 29 und die Schallquelle 7 einschließlich der Gegenmasse 27 und der Sonotrode 9 in Richtung der Aufnahme 10 verschiebt. Dadurch setzt die Sonotrode das Füllmaterial unter Druck oder verdrängt es aus dem Förderteil 6B. Insbesondere dringt die Sonotrode 9 mit ihrem freien Vorderende in die Aufnahme 10 ein und / oder kontaktiert und verschiebt einen beweglichen Stopfen des Medienbehälters 11B, wodurch das Füllmaterial aus dem Förderteil 6B gefördert wird. Die Förderung des Füllmaterials erfolgt bevorzugt zeitgleich mit der Beaufschlagung des Füllmaterials mit Schwingungen der Schallquelle 7 über die Sonotrode 9. Eine Feder 30 dient zum Rückstellen der Schiebehülse 29 und der Schallquelle 7 einschließlich der Gegenmasse 27 und der Sonotrode 9 in Richtung des Kupplungselements 5B, sobald der Anwender den Hebel 20 los lässt.

Das in der Figur 7 abgebildete Förderteil 6C wird zur Abgabe einer Spül- oder Desinfektionsflüssigkeit in einen Wurzelkanal eingesetzt. Der Aufbau der Fördervorrichtung 8 und der Schallquelle 7 gleicht jenem des Förderteils 6B der Figur 6.

Das Förderteil 6C weist einen Anschluss 10 für einen Medienbehälter oder eine Ampulle 11C auf, in der die Spül- oder Desinfektionsflüssigkeit gelagert ist. Das Werkzeug 12 ist als hohle Wurzelkanalsonde 12C ausgebildet, die mit der Fördervorrichtung 8 verbindbar ist, so dass von der Fördervorrichtung 8 geförderte Spül- oder Desinfektionsflüssigkeit durch die Sonde 12C in die Zahnwurzel abgebbar ist. Des Weiteren ist die Wurzelkanalsonde 12C mit der Schallquelle 7 verbindbar, so dass von der Schallquelle 7 erzeugte Schwingungen über die Wurzelkanalsonde 12C auf das Spül- oder Desinfektionsmittel übertragen werden, um das Spül- oder Desinfektionsmittel zu aktivieren und / oder in der Zahnwurzel zu verteilen und / oder zu durchmischen. Die Übertragung der Schwingungen von der Sonotrode 9 auf die Wurzelkanalsonde 12C erfolgt zum Beispiel über den Anschluss 10 und / oder die Ampulle 11C, insbesondere über die Ampullenwand 31 und / oder das Verbindungsstück 32 zwischen der Ampulle und der Wurzelkanalsonde 12C, und / oder über die Spül- oder Desinfektionsflüssigkeit. Die von der Schallquelle 7 erzeugten Schwingungen werden über die Sonotrode 9 auch direkt auf das Spül- oder Desinfektionsmittel übertragen.

Gemäß einem alternativen Ausführungsbeispiel weist das Förderteil 6C keine Ampulle für die Spül- oder Desinfektionsflüssigkeit auf, sondern ist mit einer Versorgungsleitung versehen, die die Spül- oder Desinfektionsflüssigkeit von einer außerhalb des Förderteils 6C angeordneten Spül- oder Desinfektionsflüssigkeitsquelle zum oder durch das Förderteil 6C leitet. Auch gemäß diesem alternativen Ausführungsbeispiel umfasst das Förderteil 6C eine Schallquelle 7, die mit der Wurzelkanalsonde 12C verbunden ist, insbesondere über die Versorgungsleitung, um Schwingungen auf die Wurzelkanalsonde 12C und die Spül- oder Desinfektionsflüssigkeit zu übertragen.

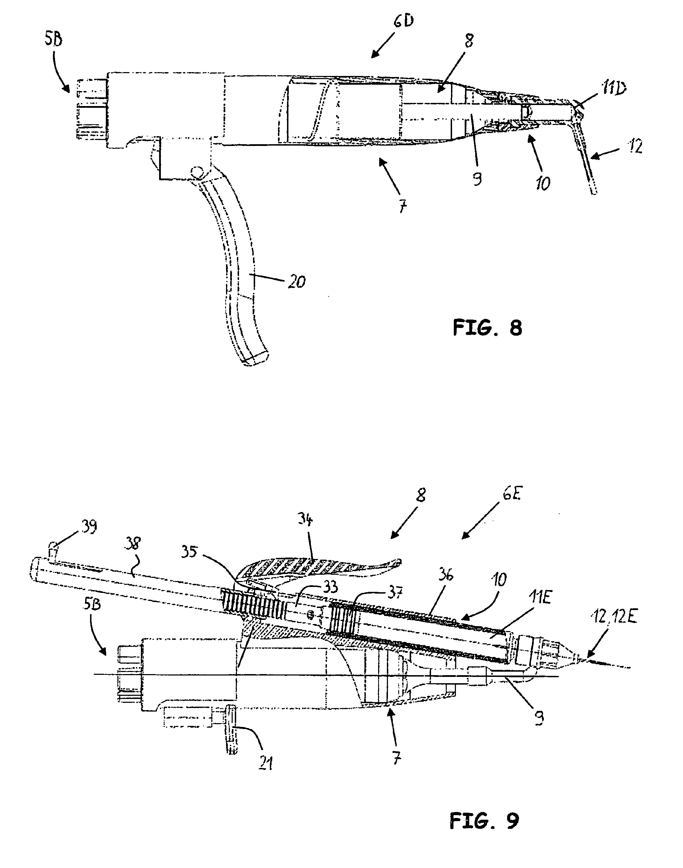

Das in der Figur 8 abgebildete Förderteil 6D dient zur Abgabe von Füllmaterial in eine Zahnwurzel, insbesondere eines Füllmaterials, bei dem durch das Beaufschlagen mit Schall oder Ultraschall und / oder Wärme eine Viskositätsänderung erzielt wird, zum Beispiel ein Zahnzement oder Gutta Percha. Der Aufbau der Fördervorrichtung 8 und der Schallquelle 7 gleicht jenem des Förderteils 6B der Figur 6, wobei insbesondere sowohl die Schwingungsübertragung als auch die Förderung des Füllmaterials durch die verschiebbare Sonotrode 9 bewirkt werden.

Der Medienbehälter 11D für das Füllmaterial ist als Kartusche ausgebildet, die ein Werkzeug 12 in Form eines länglichen, insbesondere biegbaren oder plastisch verformbaren, Röhrchens aufweist, über das das Füllmaterial in die Zahnwurzel abgegeben wird. Die Sonotrode 9 überträgt die Schwingungen der Schallquelle 7 auf das Füllmaterial, und bevorzugt direkt oder indirekt, zum Beispiel über das Füllmaterial, auch auf den Medienbehälter 11D, insbesondere auf das Röhrchen 12.

Alternativ oder zusätzlich zur Schallquelle 7 und zur Sonotrode 9 kann das Förderteil 6D zur Abgabe von Füllmaterial in eine Zahnwurzel eine Wärmequelle und einen Wärmeleiter aufweisen. Der Wärmeleiter überträgt die von der Wärmequelle erzeugte Wärme auf das Füllmaterial, wodurch dessen Viskosität verringert wird und es durch die Fördervorrichtung 8 besser förderbar ist. Die Wärmequelle umfasst bevorzugt ein elektrisches Heizelement.

Das in der Figur 9 abgebildete Förderteil 6E ist als intraossäres Handstück ausgebildet, mit dem eine Bohrung in einen Knochen, insbesondere in einen Kieferknochen, hergestellt werden kann, um ein Anästhetikum injizieren zu können. Das Förderteil 6E weist eine Außenhülse 36, eine Schallquelle 7, insbesondere eine Ultraschallquelle, und eine Sonotrode 9 auf, wobei die Sonotrode 9 nicht verschiebbar angeordnet ist. Mittels des Drückers 21 oder eines Tasters ist die Schallquelle 7 ein- und ausschaltbar. Das Förderteil 6E ist mit einem Werkzeug 12 in Form einer intraossären Nadel 12E verbindbar, wobei die Sonotrode 9 derart ausgebildet und am Förderteil 6E angeordnet ist, dass sie die von der Schallquelle 7 erzeugten Schwingungen auf die Nadel 12E überträgt. Die Sonotrode 9 kann zum Beispiel direkt die Kanüle der Nadel 12E kontaktieren oder sie überträgt die Schwingungen auf eine Kanülenschürze oder auf eine Aufnahme des Förderteils 6E, in der die Nadel 12E aufgenommen ist. Die mit einer Spitze, und insbesondere mit einer oder mehreren Schneiden, versehene Kanüle der Nadel 12E kann damit derart in Schwingung versetzt werden, dass sie, wenn sie auf einen Knochen, insbesondere einen Kieferknochen, aufgesetzt wird, in diesen eindringt und eine Bohrung erzeugt.

Des Weiteren weist das Förderteil 6E eine längliche Aufnahme 10 für einen Medienbehälter in Form einer Ampulle 11E mit dem Anästhetikum auf. Die an die Aufnahme 10 anschließende Fördervorrichtung 8 umfasst eine verschiebbare Zahnstange 33 und einen Hebel 34 mit einer Klinke 35, die mit ihrem freien Ende in die Zahnreihe der Zahnstange 33 eingreift. Die Zahnstange 33 ist von einer Schutzhülse 38 umgeben, die an einem Ende einen Schlitz aufweist, durch den ein mit der Zahnstange 33 verbundenes Griffstück 39 ragt. Bevorzugt ist an der Schutzhülse 38 eine Füllstandsanzeige für den Füllstand der Ampulle 11E vorgesehen, zum Beispiel in Form einer Skala.

Der Hebel 34 ist drehbar oder verschwenkbar am Förderteil 6E befestigt. Durch das Bewegen des Hebels 34 in Richtung der Aufnahme 10 schiebt die Klinke 35 die Zahnstange 33 in Richtung der Ampulle 11E. Berührt das Vorderende der Zahnstange 33 einen Stopfen 37 der Ampulle 11E, so verschiebt sie diesen in Richtung der Nadel 12E, so dass das Anästhetikum aus der Ampulle 11E gedrückt wird. Wird der Hebel 34 von der Aufnahme 10 wegbewegt, so wird die Klinke 35 ebenfalls nach hinten, weg von der Nadel 12E gezogen, wodurch sie an einem anderen Zahn der Zahnstange 33 eingreift.

Der Hebel 34 und der Drücker 21 sind unabhängig voneinander bedienbar, so dass mit dem Förderteil 6E entweder nur Schwingungen auf die intraossäre Nadel 12E übertragen werden oder nur Anästhetikum gefördert wird oder gleichzeitig Anästhetikum gefördert und abgegeben wird und Schwingungen auf die intraossäre Nadel 12E übertragen werden.

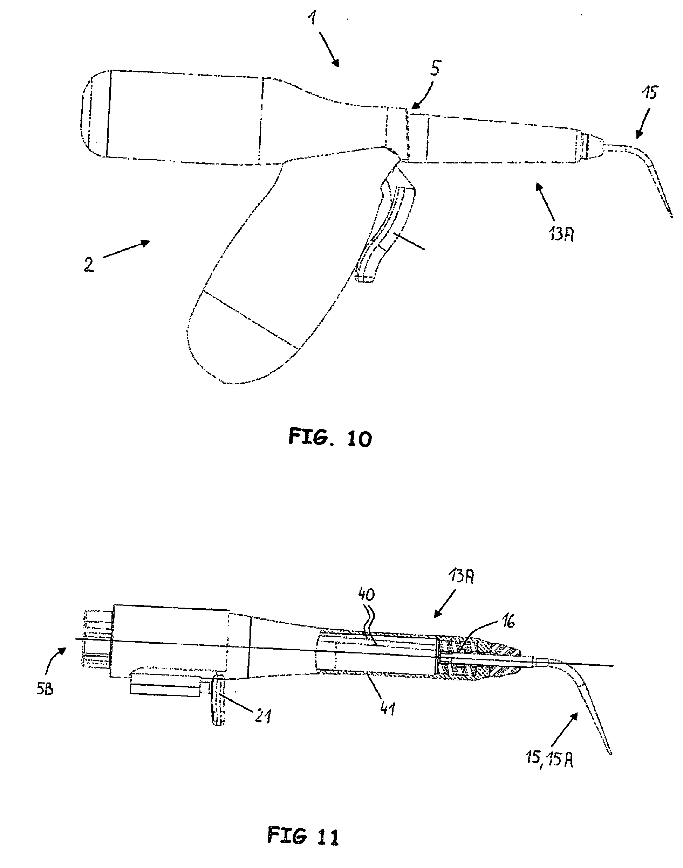

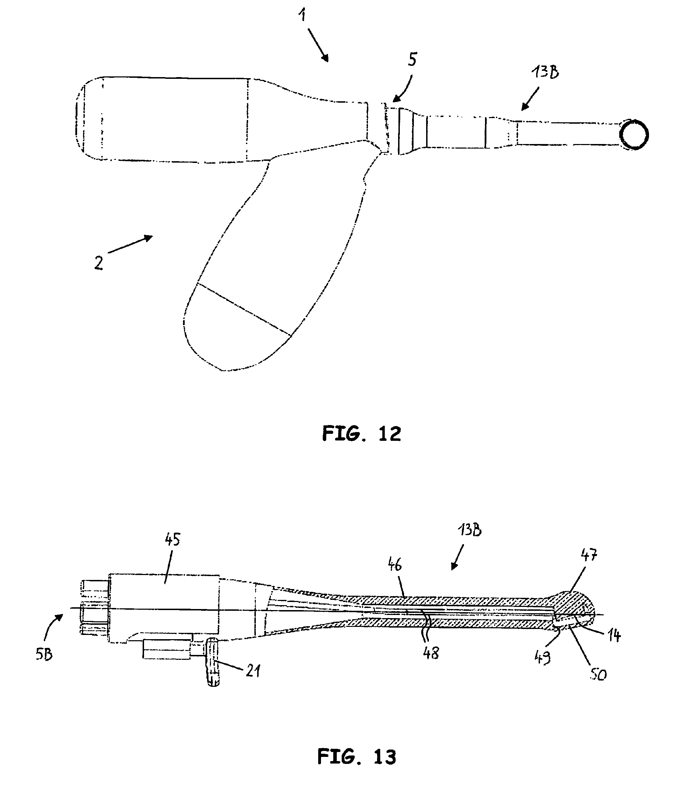

Die Figuren 10 - 16 zeigen unterschiedliche Ausführungsbeispiele von Wirkteilen 13A - 13E. Alle diese Wirkteile 13A - 13E sind über die Kupplungsvorrichtung 5, 5A, 5B mit dem Basisteil 2 aus der Figur 2 verbindbar, beispielhaft ist dies in den Figuren 10 und 12 für die Wirkteile 13A und 13B dargestellt. Je nach vorgesehener Anwendung weisen die Wirkteile 13A - 13E unterschiedliche Merkmale auf oder sind bestimmte Bauteile der Wirkteile 13A - 13E spezifisch ausgebildet. Des Weiteren lassen sich die Wirkteile 13A - 13E gemäß ihrer Anwendung in eine erste Gruppe von Wirkteilen zum Einwirken auf das von einem Förderteil 6 - 6E abgegebene Medium und eine zweite Gruppe von Wirkteilen zum Einwirken auf die Behandlungsstelle, auf die ein Förderteil 6 - 6E ein Medium abgegeben hat oder abgeben wird, unterteilen. Die erste Gruppe umfasst zum Beispiel die Wirkteile 13A, 13B, 13D, 13E, die zweite Gruppe das Wirkteil 13C.

Das in den Figuren 10 und 11 dargestellte Wirkteil 13A wird zum Obturieren von Wurzelkanälen und zum Formen, Modellieren oder Verdichten von in den Wurzelkanal abgegebenem Füllmaterial verwendet. Es weist ein Werkzeug 15 in Form einer Obturiernadel 15A auf, die lösbar oder unlösbar mit dem Wirkteil 13A verbunden ist. Zwei elektrische Leitungen 40 versorgen eine, bevorzugt im Inneren der Außenhülse 41 angeordnete, Wärmequelle 16 mit elektrischem Strom. Die Wärmequelle 16, die insbesondere ein elektrisches Heizelement umfasst, ist mit dem Werkzeug 15 verbunden oder als Teil des Werkzeugs 15 ausgebildet, so dass durch die Wärmequelle 16 das Werkzeug 15 erwärmbar ist. Das Werkzeug 15 überträgt die Wärme auf das Füllmaterial, wodurch dessen Viskosität sinkt. Mittels eines Tasters oder des Drückers 21 kann die Wärmequelle 16 ein- und ausgeschaltet werden, bevorzugt kann damit auch die Temperatur der Obturiernadel 15A vorgegeben werden.

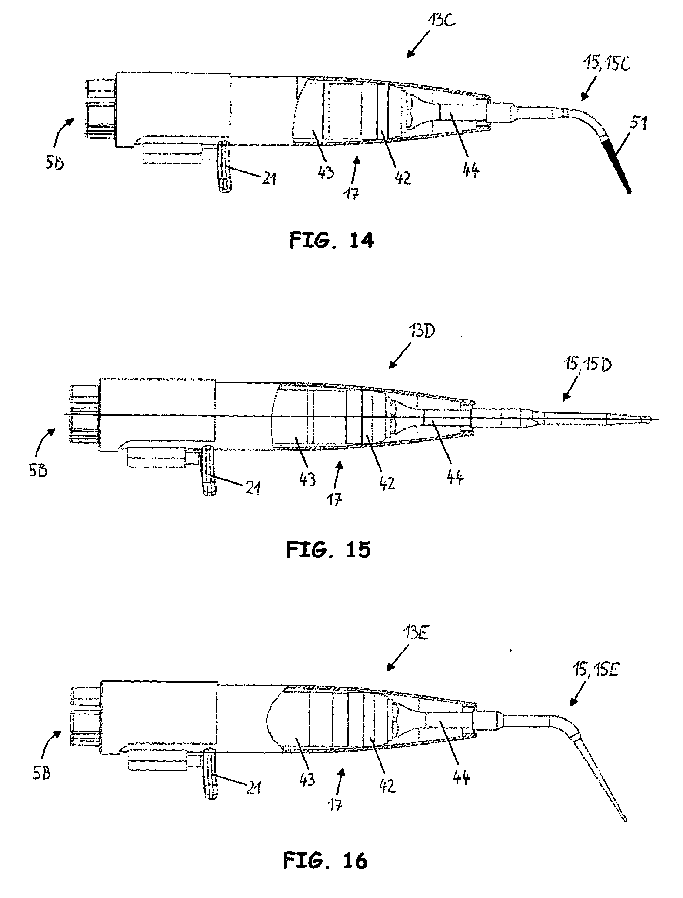

Das in der Figur 16 dargestellte Wirkteil 13E dient ebenfalls zum Obturieren von Wurzelkanälen und zum Formen, Modellieren oder Verdichten von in den Wurzelkanal abgegebenem Füllmaterial. Es weist eine Schallquelle 17, insbesondere eine Ultraschallquelle, auf. Die Schallquelle 17 ist gemäß einem Ausführungsbeispiel gleich aufgebaut wie Schallquelle 7 der Förderteil 6 - 6E und umfasst einen piezoelektrischen, magnetostriktiven oder pneumatischen Schallwandler 42, ein Gegenmasse 43 und eine Sonotrode 44. Die Sonotrode 44 ist lösbar oder unlösbar mit dem Werkzeug 15, insbesondere einer Obturiernadel 15E, verbunden, so dass die von der Schallquelle 17 erzeugten Schwingungen auf das Werkzeug 15 und auf das Füllmaterial übertragbar sind. Durch die Beaufschlagung des Füllmaterials wird gemäß einer bevorzugten Ausgestaltung dessen Viskosität erniedrigt. Mittels eines Tasters oder des Drückers 21 kann die Schallquelle 17 ein- und ausgeschaltet werden, bevorzugt kann damit auch zumindest ein Betriebsparameter der Schallquelle 17, zum Beispiel die Frequenz oder die Leistung, vorgegeben werden.

Selbstverständlich ist es auch möglich ein Wirkteil zum Obturieren von Wurzelkanälen und zum Formen, Modellieren oder Verdichten von in den Wurzelkanal abgegebenem Füllmaterial zu schaffen, das eine Kombination der beiden Wirkteile 13A und 13E ist und eine Schallquelle 17 und eine Wärmequelle 16 aufweist.

Das in den Figuren 12 und 13 dargestellte Wirkteil 13B wird zur Abgabe von elektromagnetischer Strahlung verwendet, insbesondere zur Abgabe von Strahlung auf eine Füllmasse auf Kunstharzbasis, die durch das Einwirken von Licht polymerisierbar oder aushärtbar ist, insbesondere eine hochviskose, einen hohen Anteil an anorganischen Füllstoffen aufweisende dentalen Füllmasse auf Kunstharzbasis.

Das Wirkteil 13B weist einen Anschlussteil 45, einen länglichen Halsteil 46 und einen daran anschließenden Kopfteil 47 auf. Im Inneren des Wirkteils 13B, insbesondere im Kopfteil 47, ist eine Strahlungsquelle 14 vorgesehen. Bevorzugt weist die Strahlungsquelle 14 eine oder mehrere optische Halbleiterelement oder Leuchtdioden (LEDs) auf. Zwei elektrische Leitungen 48 versorgen die Strahlungsquelle 14 mit elektrischer Energie. Der Kopfteil 47 weist eine Strahlungsabgabeöffnung 49 auf, durch die die von der Strahlungsquelle 14 erzeugte Strahlung abgegeben wird und die bevorzugt durch ein transparentes Schutzglas 50 verschlossen ist. Im Wirkteil 13B können weitere optische Bauteile wie zum Beispiel Linsen, Spiegel, Strahlungsleiter oder Reflektoren für die zu emittierende Strahlung vorgesehen sein. Ein Taster oder der Drücker 21 dient zum Ein-/Ausschalten der Strahlungsquelle 14.

Alternativ ist es auch möglich, dass die Strahlungsquelle außerhalb des Wirkteils angeordnet ist und das Wirkteil somit keine Strahlungsquelle und keine elektrische Leitungen zur Versorgung der Strahlungsquelle aufweist. Stattdessen ist in diesem Wirkteil ein Strahlengang und / oder ein Strahlungsleiter vorgesehen, der sich von dem Kupplungselement 5B, an dem die Strahlung eingekoppelt wird, bis zur Strahlungsabgabeöffnung im Kopfteil erstreckt und die Strahlung durch das Wirkteil leitet.

Die Wirkteile 13C und 13D gleichen in ihrem Aufbau dem Wirkteil 13E der Figur 16 und weisen wiederum eine Schallquelle 17, insbesondere eine Ultraschallquelle, und einen Taster oder Drücker 21 auf. Die Werkzeuge 15 der beiden Wirkteile 13C, 13D sind mit der Schallquelle 17 verbindbar oder verbunden, so dass Schwingungen auf sie übertragbar sind.

Das Wirkteil 13D dient zum Formen, Modellieren oder Verdichten von in eine, insbesondere dentale, Kavität abgegebenem Füllmaterial. Es ist mit einem als Modelliersonde 15D ausgebildeten Werkzeug 15 lösbar oder unlösbar verbunden. Die von der Schallquelle 17 erzeugten Schwingungen werden über das Werkzeug 15 auf das Füllmaterial übertragen, wodurch dessen Viskosität sinkt.

Das mit dem Wirkteil 13C lösbar oder unlösbar verbundene endodontische Werkzeug 15 in Form einer endodontischen Nadel oder Dentalfeile 15C dient zum Erweitern eines Zahnwurzelkanals. Die Dentalfeile 15C weist dazu einen abrasiven Abschnitt 51 auf, der durch die Schallquelle 17 in Schwingung versetzt wird und der aufgrund seiner Schwingungsbewegung Gewebe des Zahnwurzelkanals abträgt.

Bevorzugt sind die Förderteile 6 - 6E und / oder die Wirkteile 13A - 13E, insbesondere ihre Außenhülsen, aus Materialien gefertigt, die negativen Einflüssen durch Sterilisation oder Thermodesinfektion, wie zum Beispiel Korrosion, widerstehen.

Die Erfindung ist nicht auf die beschriebenen Ausführungsbeispiele beschränkt, sondern umfasst alle Ausführungen und Anwendungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

So können insbesondere unterschiedliche Anwendungen durch ein einziges Förderteil 6 - 6E oder Wirkteil 13A - 13E durchgeführt werden, wenn dieses mit unterschiedlichen Aufnahmen 10, Medienbehältern 11 und / oder Werkzeugen 15 verbindbar ist. So ist es zum Beispiel denkbar, dass an ein einziges Förderteil Medienbehälter mit unterschiedlichen Medien, zum Beispiel mit einem Füllmaterial für eine Kavität und mit einem Füllmaterial für einen Wurzelkanal, anschließbar sind, so dass dieses Förderteil zur Abgabe von unterschiedlichen Medien und / oder zur Abgabe eines oder unterschiedlicher Medien an unterschiedliche Behandlungsort verwendbar ist. Entsprechendes gilt für die Wirkteile, wobei zum Beispiel ein einziges Wirkteil, das eine Schallquelle umfasst, mit einer Feile 15C, einer Modelliersonde 15D oder einer Obturiernadel 15E verbindbar ist.

Es sei des Weiteren darauf hingewiesen, dass die Behandlungsvorrichtung 1 gemäß einem Ausführungsbeispiel ein Basisteil 2 sowie alle in den Abbildungen 1 - 16 dargestellten Förderteile 6 - 6E oder Wirkteile 13A - 13E umfasst. Gemäß einem anderen Ausführungsbeispiel umfasst die Behandlungsvorrichtung 1 nicht alle dieser Förderteile 6 - 6E oder Wirkteile 13A - 13E, sondern weniger Förderteile 6 - 6E und / oder Wirkteile 13A - 13E, insbesondere zumindest ein Förderteil 6 - 6E und ein weiteres Förderteil 6 - 6E oder ein Wirkteil 13A - 13E.

## Patentansprüche

1. Medizinische, insbesondere dentale, Behandlungsvorrichtung (1) zur Abgabe eines Mediums, wobei die Behandlungsvorrichtung (1) als kabelloses Handstück ausgebildet ist, umfassend ein Basisteil (2) mit einer Energieversorgungseinheit (3), welche als Batterie oder Akkumulator ausgebildet ist, und einer elektrischen oder elektronischen Steuervorrichtung (4), **gekennzeichnet durch**
zumindest ein mit dem Basisteil (2) über eine Kupplungsvorrichtung (5, 5A, 5B) lösbar verbindbares Förderteil (6 - 6E), das eine Schallquelle (7), insbesondere eine Ultraschallquelle, eine Fördervorrichtung (8) zur Abgabe des Mediums von dem Förderteil (6 - 6E), eine Aufnahme für das Medium oder einen Anschluss (10) für einen Medienbehälter (11B - 11E) und eine Sonotrode (9) zur Übertragung der von der Schallquelle (7), insbesondere der Ultraschallquelle, erzeugten Schwingungen auf das Medium, die Aufnahme und / oder den Anschluss (10) umfasst.

2. Medizinische, insbesondere dentale, Behandlungsvorrichtung (1) nach Anspruch 1,
**dadurch gekennzeichnet, dass**
das zumindest eine Förderteil (6 - 6E) eine Strahlungsquelle zur Abgabe einer das Medium aktivierenden Strahlung aufweist.

3. Medizinische, insbesondere dentale, Behandlungsvorrichtung (1) nach einem der vorherstehenden Ansprüche, **dadurch gekennzeichnet, dass**
das zumindest eine Förderteil (6 - 6E) ein Werkzeug (12) für das Medium, insbesondere eine Modelliersonde, eine intraossäre Nadel (12E) oder eine Wurzelkanalsonde (12C), und eine Sonotrode (9) zur Übertragung der von der Schallquelle (7), insbesondere der Ultraschallquelle, erzeugten Schwingungen auf das Werkzeug (12) umfasst.

4. Medizinische, insbesondere dentale, Behandlungsvorrichtung (1) nach einem der vorherstehenden Ansprüche, **dadurch gekennzeichnet, dass**
das zumindest eine Förderteil (6 - 6E) eine Wärmequelle zur Erwärmung des Mediums aufweist.

5. Medizinische, insbesondere dentale, Behandlungsvorrichtung (1) nach einem der vorherstehenden Ansprüche, **gekennzeichnet durch**
zumindest ein mit dem Basisteil (2) über die Kupplungsvorrichtung (5, 5A, 5B) lösbar verbindbares Wirkteil (13A, 13B, 13D, 13E) zum Einwirken auf das vom Förderteil (6 - 6E) abgegebene Medium.

6. Medizinische, insbesondere dentale, Behandlungsvorrichtung (1) nach Anspruch 5, **dadurch gekennzeichnet, dass**
das zumindest eine Wirkteil (13A, 13B, 13D, 13E) eine Strahlungsquelle (14) zur Abgabe einer das Medium aktivierenden Strahlung aufweist.

7. Medizinische, insbesondere dentale, Behandlungsvorrichtung (1) nach einem der Ansprüche 5 - 6, **dadurch gekennzeichnet, dass**
das zumindest eine Wirkteil (13A, 13B, 13D, 13E) ein Werkzeug (15) für das abgegebene Medium umfasst, insbesondere eine Modelliersonde (15D) oder eine Obturiernadel (15A).

8. Medizinische, insbesondere dentale, Behandlungsvorrichtung (1) nach Anspruch 7,
**dadurch gekennzeichnet, dass**
das zumindest eine Wirkteil (13A, 13B, 13D, 13E) eine Schallquelle (17), insbesondere eine Ultraschallquelle, zur Übertragung von Schwingungen auf das Werkzeug (15) aufweist.

9. Medizinische, insbesondere dentale, Behandlungsvorrichtung (1) nach einem der Ansprüche 5 - 8, **dadurch gekennzeichnet, dass**
das zumindest eine Wirkteil (13A, 13B, 13D, 13E) eine Wärmequelle (16) zur Erwärmung des Mediums aufweist.

10. Medizinische, insbesondere dentale, Behandlungsvorrichtung (1) nach einem der vorherstehenden Ansprüche, **gekennzeichnet durch**
zumindest ein mit dem Basisteil (2) über die Kupplungsvorrichtung (5, 5A, 5B) lösbar verbindbares Wirkteil (13C) zum Einwirken auf die Behandlungsstelle, auf die das Förderteil (6 - 6E) das Medium abgegeben hat oder abgeben wird.

11. Medizinische, insbesondere dentale, Behandlungsvorrichtung (1) nach Anspruch 10, **dadurch gekennzeichnet, dass**
das zumindest eine Wirkteil (13C) ein Werkzeug (15C) zur Wurzelkanalaufbereitung und bevorzugt eine Schallquelle (17), insbesondere eine Ultraschallquelle, zur Übertragung von Schwingungen auf das Werkzeug (15C) aufweist.

12. Medizinische, insbesondere dentale, Behandlungsvorrichtung (1) nach einem der vorherstehenden Ansprüche, **dadurch gekennzeichnet, dass**
das Basisteil (2) einen, insbesondere pistolenförmigen, Griffabschnitt aufweist, in dem zumindest ein Teil der Energieversorgungseinheit (3) und / oder der elektrischen oder elektronischen Steuervorrichtung (4) aufgenommen ist.

13. Medizinische, insbesondere dentale, Behandlungsvorrichtung (1) nach einem der vorherstehenden Ansprüche, **dadurch gekennzeichnet, dass**
die Schallquelle (7) als piezoelektrische Schallquelle oder als magnetostriktive Schallquelle ausgebildet ist.

14. Medizinische, insbesondere dentale, Behandlungsvorrichtung (1) nach einem der vorherstehenden Ansprüche, **dadurch gekennzeichnet, dass**
die Behandlungsvorrichtung (1) mehrere Förderteile mit unterschiedlichen Schallquellen aufweist.

15. Medizinische, insbesondere dentale, Behandlungsvorrichtung (1) nach einem der vorherstehenden Ansprüche, **dadurch gekennzeichnet, dass**
die elektrische oder elektronische Steuervorrichtung (4) ausgebildet ist, die an das Basisteil (2) anschließbaren Förderteile (6 - 6E) oder Wirkteile (13A - 13E) zu erkennen und für das jeweilige angeschlossene Förderteil (6 - 6E) oder Wirkteil (13A - 13E) zumindest einen spezifischen Betriebsparameter auszuwählen und das angeschlossene Förderteil (6 - 6E) oder Wirkteil (13A - 13E) gemäß diesem Betriebsparameter zu betreiben.

## Claims

1. A medical, in particular dental, treatment device (1) for dispensing a medium, wherein the treatment device (1) is designed as a wireless handpiece, comprising a base part (2) with a power supply unit (3), which is designed as a battery or an accumulator, and an electrical or electronic control device (4), **characterized by** at least one delivery part (6 - 6E), which can be detachably connected to the base part (2) via a coupling device (5, 5A, 5B) and which comprises a sound source (7), in particular an ultrasound source, a delivery device (8) for dispensing the medium from the delivery part (6 - 6E), a receptacle for the medium or a connection (10) for a media container (11B - 11E) and a sonotrode (9) for transferring the vibrations generated by the sound source (7), in particular the ultrasound source, to the medium, the receptacle and/or the connection (10).

2. The medical, in particular dental, treatment device (1) according to Claim 1,
**characterized in that**
the at least one delivery part (6 - 6E) comprises a radiation source for emitting a radiation which activates the medium.

3. The medical, in particular dental, treatment device (1) according to any one of the preceding claims, **characterized in that**
the at least one delivery part (6 - 6E) comprises a tool (12) for the medium, in particular a modeling probe, an intraosseous needle (12E) or a root canal probe (12C) and a sonotrode (9) for transferring the vibrations generated by the sound source (7), in particular the ultrasound source, to the tool (12).

4. The medical, in particular dental, treatment device (1) according to any one of the preceding claims, **characterized in that**
the at least one delivery part (6 - 6E) comprises a heat source for heating the medium.

5. The medical, in particular dental, treatment device (1) according to any one of the preceding claims, **characterized by**
at least one active part (13A, 13B, 13D, 13E), which is detachably connected to the base part (2) via the coupling device (5, 5A, 5B) for acting on the medium dispensed by the delivery part (6 - 6E).

6. The medical, in particular dental, treatment device (1) according to Claim 5, **characterized in that**
the at least one active part (13A, 13B, 13D, 13E) comprises a radiation source (14) for emitting a radiation that activates the medium.

7. The medical, in particular dental, treatment device (1) according to any one of Claims 5 - 6, **characterized in that**
the at least one active part (13A, 13B, 13D, 13E) comprises a tool (15) for the medium dispensed, in particular a modeling probe (15D) or an obturation needle (15A).

8. The medical, in particular dental, treatment device (1) according to Claim 7, **characterized in that**
the at least one active part (13A, 13B, 13D, 13E) comprises a sound source (17), in particular an ultrasound source, for transferring vibrations to the tool (15).

9. The medical, in particular dental, treatment device (1) according to any one of Claims 5 - 8, **characterized in that**
the at least one active part (13A, 13B, 13D, 13E) comprises a heat source (16) for heating the medium.

10. The medical, in particular dental, treatment device (1) according to any one of the preceding claims, **characterized by**
at least one active part (13C) detachably connectable to the base part (2) via the coupling device (5, 5A, 5B) for acting on the treatment site to which the delivery part (6 - 6E) has dispensed or will dispense the medium.

11. The medical, in particular dental, treatment device (1) according to Claim 10, **characterized in that**
the at least one active part (13C) comprises a tool (15C) for preparing the root canal and preferably comprises a sound source (17), in particular an ultrasound source, for transferring vibrations to the tool (15C).

12. The medical, in particular dental, treatment device (1) according to any one of the preceding claims, **characterized in that**
the base part (2) comprises a, in particular pistol-shaped, handle section in which at least a part of the power supply unit (3) and/or the electrical or electronic control device (4) is held.

13. The medical, in particular dental, treatment device (1) according to any one of the preceding claims, **characterized in that**
the sound source (7) is designed as a piezoelectric sound source or as a magnetostrictive sound source.

14. The medical, in particular dental, treatment device (1) according to any one of the preceding claims, **characterized in that**
the treatment device (1) comprises multiple delivery parts having different sound sources.

15. The medical, in particular dental, treatment device (1) according to any one of the preceding claims, **characterized in that**
the electrical or electronic control device (4) is designed to recognize delivery parts (6 - 6E) or active parts (13A - 13E) that can be connected to the base part (2) and to select at least one specific operating parameter for the respective connected delivery part (6 - 6E) or active part (13A -13E) and to operate the connected delivery part (6 - 6E) or active part (13A - 13E) according to this operating parameter.

## Revendications

1. Dispositif de traitement médical (1), en particulier dentaire, pour délivrer un milieu, dans lequel le dispositif de traitement (1) est réalisé sous la forme d'une pièce à main sans fil, comprenant une partie de base (2) avec une unité d'alimentation en énergie (3), réalisée sous forme de batterie ou d'accumulateur, et un dispositif de commande électrique ou électronique (4), **caractérisé par**
au moins une partie de transport (6 - 6E) pouvant être reliée de façon amovible à la partie de base (2) par l'intermédiaire d'un dispositif d'accouplement (5, 5A, 5B) et comprenant une source sonore (7), en particulier une source ultrasonore, un dispositif de transport (8) pour délivrer le milieu à partir de la partie de transport (6 - 6E), un réservoir pour le milieu ou un raccord (10) pour un récipient de milieu (11B - 11E) et une sonotrode (9) pour transmettre les vibrations produites par la source sonore (7), en particulier la source ultrasonore, au milieu, au réservoir et/ou au raccord (10).

2. Dispositif de traitement médical (1), en particulier dentaire, selon la revendication 1, **caractérisé en ce que**
ladite au moins une partie de transport (6 - 6E) présente une source de rayonnement pour délivrer un rayonnement activant le milieu.

3. Dispositif de traitement médical (1), en particulier dentaire, selon l'une quelconque des revendications précédentes, **caractérisé en ce que**
ladite au moins une partie de transport (6 - 6E) comprend un outil (12) pour le milieu, en particulier une sonde de modelage, une aiguille intra-osseuse (12E) ou une sonde à canaux radiculaires (12C), et une sonotrode (9) pour transmettre à l'outil (12) les vibrations produites par la source sonore (7), en particulier la source ultrasonore.

4. Dispositif de traitement médical (1), en particulier dentaire, selon l'une quelconque des revendications précédentes, **caractérisé en ce que**
ladite au moins une partie de transport (6 - 6E) présente une source de chaleur pour chauffer le milieu.

5. Dispositif de traitement médical (1), en particulier dentaire, selon l'une quelconque des revendications précédentes, **caractérisé par**
au moins une partie active (13A, 13B, 13D, 13E) pouvant être reliée de façon amovible à la partie de base (2) par l'intermédiaire du dispositif d'accouplement (5, 5A, 5B) pour agir sur le milieu délivré par la partie de transport (6 - 6E).

6. Dispositif de traitement médical (1), en particulier dentaire, selon la revendication 5, **caractérisé en ce que**
ladite au moins une partie active (13A, 13B, 13D, 13E) présente une source de rayonnement (14) pour délivrer un rayonnement activant le milieu.

7. Dispositif de traitement médical (1), en particulier dentaire, selon l'une quelconque des revendications 5 et 6, **caractérisé en ce que**
ladite au moins une partie active (13A, 13B, 13D, 13E) comprend un outil (15) pour le milieu délivré, en particulier une sonde de modelage (15D) ou une aiguille d'obturation (15A).

8. Dispositif de traitement médical (1), en particulier dentaire, selon la revendication 7, **caractérisé en ce que**
ladite au moins une partie active (13A, 13B, 13D, 13E) présente une source sonore (17), en particulier une source ultrasonore, pour transmettre des vibrations à l'outil (15).

9. Dispositif de traitement médical (1), en particulier dentaire, selon l'une quelconque des revendications 5 à 8, **caractérisé en ce que**
ladite au moins une partie active (13A, 13B, 13D, 13E) présente une source de chaleur (16) pour chauffer le milieu.

10. Dispositif de traitement médical (1), en particulier dentaire, selon l'une quelconque des revendications précédentes, **caractérisé par**
au moins une partie active (13C) pouvant être reliée de façon amovible à la partie de base (2) par l'intermédiaire du dispositif d'accouplement (5, 5A, 5B) pour agir sur le site à traiter, où la partie de transport (6 - 6E) a distribué ou distribuera le milieu.

11. Dispositif de traitement médical (1), en particulier dentaire, selon la revendication 10, **caractérisé en ce que**
ladite au moins une partie active (13C) présente un outil (15C) pour le traitement des canaux radiculaires et présente de préférence une source sonore (17), en particulier une source ultrasonore, pour transmettre des vibrations à l'outil (15C).

12. Dispositif de traitement médical (1), en particulier dentaire, selon l'une quelconque des revendications précédentes, **caractérisé en ce que**
la partie de base (2) présente une section de poignée, en particulier en forme de pistolet, qui reçoit au moins une partie de l'unité d'alimentation en énergie (3) et/ou du dispositif de commande électrique ou électronique (4).

13. Dispositif de traitement médical (1), en particulier dentaire, selon l'une quelconque des revendications précédentes, **caractérisé en ce que**
la source sonore (7) est réalisée sous forme de source sonore piézoélectrique ou de source sonore magnétostrictive.

14. Dispositif de traitement médical (1), en particulier dentaire, selon l'une quelconque des revendications précédentes, **caractérisé en ce que**
le dispositif de traitement (1) présente plusieurs parties de transport avec différentes sources sonores.

15. Dispositif de traitement médical (1), en particulier dentaire, selon l'une quelconque des revendications précédentes, **caractérisé en ce que**
le dispositif de commande électrique ou électronique (4) est réalisé pour reconnaître les parties de transport (6 - 6E) ou les parties actives (13A - 13E) pouvant être raccordées à la partie de base (2) et pour sélectionner pour la partie de transport (6 - 6E) ou la partie active (13A - 13E) respectivement raccordée au moins un paramètre d'opération spécifique et pour actionner la partie de transport (6 - 6E) ou la partie active (13A - 13E) selon ce paramètre d'opération.
